Europäisches Patentamt

European Patent Office

Office européen des brevets

(11) Numéro de publication: **0 253 688 B1**

(19)

# (12) FASCICULE DE BREVET EUROPEEN

(45) Date de publication de fascicule du brevet: **15.07.92**　(51) Int. Cl.⁵: **A61B 17/16**, A61F 2/08

(21) Numéro de dépôt: **87400778.4**

(22) Date de dépôt: **07.04.87**

(54) Instrument chirurgical de visée et de pose du ligament croisé postérieur du genou.

(30) Priorité: **07.05.86 FR 8606656**

(43) Date de publication de la demande:
**20.01.88 Bulletin 88/03**

(45) Mention de la délivrance du brevet:
**15.07.92 Bulletin 92/29**

(84) Etats contractants désignés:
**AT BE CH DE ES GB GR IT LI LU NL SE**

(56) Documents cités:
**EP-A- 0 153 831**
**DE-A- 2 747 568**
**FR-A- 820 187**

(73) Titulaire: **Laboureau, Jacques-Philippe**
**24 rue de la Fontaine Billenois**
**F-21000 Dijon(FR)**

(72) Inventeur: **Laboureau, Jacques-Philippe**
**24 rue de la Fontaine Billenois**
**F-21000 Dijon(FR)**

(74) Mandataire: **Bruder, Michel**
**Cabinet Michel Bruder Conseil en Brevets**
**10, rue de la Pépinière**
**F-75008 Paris(FR)**

Rank Xerox (UK) Business Services

# Description

La présente invention concerne un instrument chirurgical de visée et de pose pour la plastie (ou remplacement prothétique) du ligament croisé postérieur du genou (LCP).

Lors d'une rupture ligamentaire consécutive, par exemple, à un accident, il est bien connu qu'il peut être nécessaire de procéder au remplacement du ou des ligaments endommagés qui, pour certains d'entre eux, ne peuvent se reconstituer d'eux-mêmes : c'est notamment le cas du LCP du genou.

La difficulté du remplacement d'un tel ligament n'est plus liée au ligament lui-même, puisque l'on connaît maintenant des réalisations tout à fait convenables de ligaments artificiels. En revanche, la chirurgie attachée à des opérations de ce type présente de nombreux inconvénients, particulièrement liés à des problèmes anatomiques.

En effet, pour remplacer un LCP, il faut naturellement intervenir sur le condyle fémoral, donc en avant du genou du patient, mais aussi sur la face postérieure du tibia, c'est-à-dire en arrière du genou du patient. Tout ceci suppose donc de difficiles manipulations du même patient en situation d'opéré, sur une table chirurgicale, dans un environnement où les notions d'espace et de temps peuvent rapidement devenir cruciales. Un instrument selon le préambule de la revendication 1 est connu du document DE-A-2 747 568.

La présente invention vise à remédier à ces inconvénients en procurant un instrument chirurgical de visée et de pose pour la plastie (ou remplacement prothétique) du ligament croisé postérieur du genou comportant une partie qui puisse être introduite au travers de l'échancrure fémorale et un dispositif de visée, caractérisé en ce que pour permettre d'effectuer cette chirurgie par un seul abord antérieur, tout en obtenant un point de pénétration anatomique dudit LCP à la face postérieure du tibia et éviter toute blessure des éléments postérieurs notamment vasculaires l'instrument comprend :

d'une part, une gouge composée d'un manche et d'une lame en forme de rugine en "S" italique, dont la partie terminale interne est de section concave, telle que ladite lame puisse être introduite au travers de ladite échancrure fémorale jusqu'à la face postérieure du tibia,

et d'autre part, ledit dispositif de visée, qui vient se solidariser à la gouge par des moyens de fixation rapide, et comprend à son extrêmité, disposés en regard et à distance de la partie concave de la lame, deux canons de visée accolés en direction de ladite partie concave suivant une obliquité telle que l'angle formé par le prolongement de l'axe des canons et la tangente à la face interne de la partie concave de la lame, en leur point de rencontre, soit supérieur à 90°, la longueur desdits canons de visée étant suffisante pour assurer le guidage longitudinal de forets utilisés pour la perforation transversale de deux tunnels dans la partie tibiale, ceci permettant la mise en place simultanée de deux faisceaux postéro-interne et postéro-externe du LCP.

L'instrument suivant l'invention peut comporter, dans la partie terminale de la gouge, un bord tranchant en forme de biseau venant, par raclage le long de la face postérieure du tibia, désinsérer les éléments attachés audit tibia, et ce sur une hauteur déterminée par un repère gravé à l'intérieur de la partie concave de la lame de la gouge, ce repère étant visualisé par l'opérateur dans l'espace intra-articulaire ; dès lors que le repère est en coïncidence avec le haut du tibia, le dégagement de la face postérieure du tibia est alors suffisant pour effectuer la perforation de deux tunnels destinés à recevoir ultérieurement, pour les fixer, les faisceaux de la prothèse ligamentaire, préalablement repliée en forme de "U". La gouge, toujours en position intra-articulaire, le bord tranchant en appui sur la face postérieure du tibia, reçoit alors la pièce "porte-canons" en avant du tibia du patient ; les deux canons accolés se trouvent ainsi placés perpendiculairement à la face antérieure du tibia, suivant des axes colinéaires dont les prolongements aboutissent chacun à l'intérieur de la partie concave de la gouge et présentent une obliquité dans le plan vertical telle que l'angle formé entre eux et la tangente à la lame de gouge en leur point de rencontre, soit obtus.

Ainsi disposé, l'instrument permet d'effectuer, par le moyen d'une perceuse munie de son foret, la perforation des deux tunnels trans-tibiaux avec les avantages corrélatifs suivants :
- le foret est parfaitement guidé dans la masse osseuse ;
- lorsque ledit foret débouche en partie postérieure du tibia, celui-ci ne peut occasionner de blessure au paquet vasculaire qui est protégé par la face concave de la lame de gouge;
- l'axe de chacun des tunnels ainsi obtenu présente une obliquité facilitant grandement le procédé de mise en place de la prothèse.

Après retrait du matériel de perforation, l'instrument au complet restant en place, éventuellement maintenu par une broche en acier passée dans un oeillet prévu à cet effet, juste au-dessus des canons de visée et enfoncée dans l'os, on introduit dans les tunnels deux broches souples munies, à leurs extrémités, d'un chas jusqu'à ce qu'elles rencontrent la partie concave de la lame de la gouge, suivant un anble obtus imposant auxdites broches le seul moyen de remonter à l'intérieur de la lame suivant deux sillons parallèles s'étendant au moins depuis le point de rencontre broche-lame jusqu'à 10 mm environ au-dessus du repère de positionnement déjà cité.

Lorsque les broches souples apparaissent dans l'échancrure inter-condylienne, on peut les saisir pour y attacher les deux extrémités du ligament mis en forme de "U", préalablement enfilées dans deux tunnels forés à partir d'un point unique dans l'échancrure inter-condylienne formant le point anatomique d'insertion fémorale du LCP et débouchant en extra-synovial, au-dessus de la joue condylienne, suivant deux orifices distants d'un centimètre environ permettant la retenue instantanée du fond du "U" du ligament.

Il reste ensuite à retirer les deux broches souples suivant un chemin inverse, ce qui procure l'avantage d'enfiler automatiquement les deux faisceaux ligamentaires d'arrière en avant dans les deux tunnels trans-tibiaux.

Ainsi récupérés sur la face antérieure du tibia, les faisceaux sont suffisamment tendus pour que la position tiroir postérieur soit égale à zéro ; les deux extrémités desdits faisceaux sont alors amarrées sur le tibia par deux agrafes spécialisées.

On décrira ci-après, à titre d'exemple non limitatif, une forme d'exécution de la présente invention, en référence au dessin annexé sur lequel :

La figure 1 est une vue en élévation de l'instrument chirurgical complet suivant l'invention, comportant la gouge et l'ensemble de visée ;

Les figures 2 et 3 sont respectivement des vues de face de la gouge seule présentant la partie interne et concave de la lame et une vue de côté de la même gouge ;

Les figures 4 et 5 sont respectivement une vue de face de l'instrument comportant les canons de visée et une vue de côté du même instrument ;

La figure 6 est une vue en perspective de l'articulation fémur/tibia montrant, mise en place dans l'échancrure intercondylienne, de la lame de la gouge amenée dans sa position de travail ;

La figure 7 est une vue en coupe longitudinale du genou présentant l'instrument chirurgical complet suivant l'invention en position effective de travail ;

La figure 8 est une vue en coupe longitudinale du genou semblable à celle de la figure 7, montrant la mise en place des faisceaux du ligament croisé postérieur (LCP) ;

La figure 9 est une vue en perspective de l'articulation du genou munie de son LCP artificiel dans sa diposition finale.

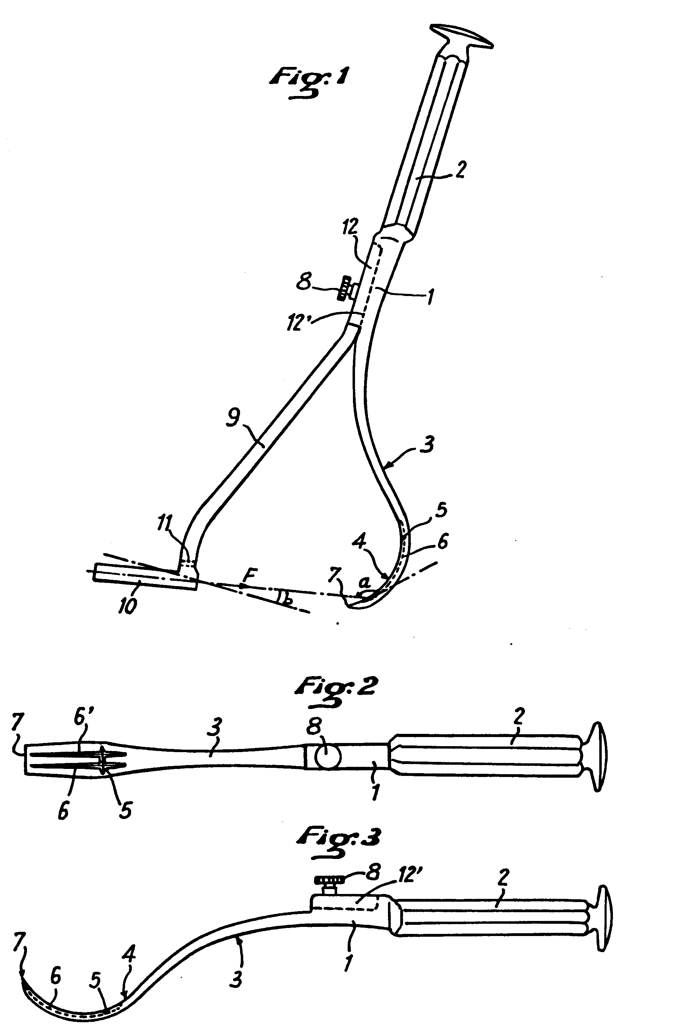

L'instrument chirurgical suivant l'invention, conformément à la figure 1, est composé de deux parties fonctionnelles principales :

- une gouge 1 comportant un manche 2 assurant la manipulation d'une lame en forme de rugine doublement incurvée suivant un "S" italique; le manche 2 étant fixé en bout de la partie supérieure 3 du "S", la partie inférieure 4 présentant sur toute sa face interne une concavité analogue à une cuillère allongée ;

- un dispositif de visée 9, comportant deux canons 10 et 10′ accolés, fixé, au moment voulu, à la gouge 1 par emboîtement de sa partie terminale 12 dans une rainure 12′ prévue à cet effet dans le manche 2 de la gouge 1, l'ensemble étant solidarisé par une vis à tête moletée 8, en sorte que la position relative du dispositif 9 et de la gouge 1 présente toujours une bonne précision.

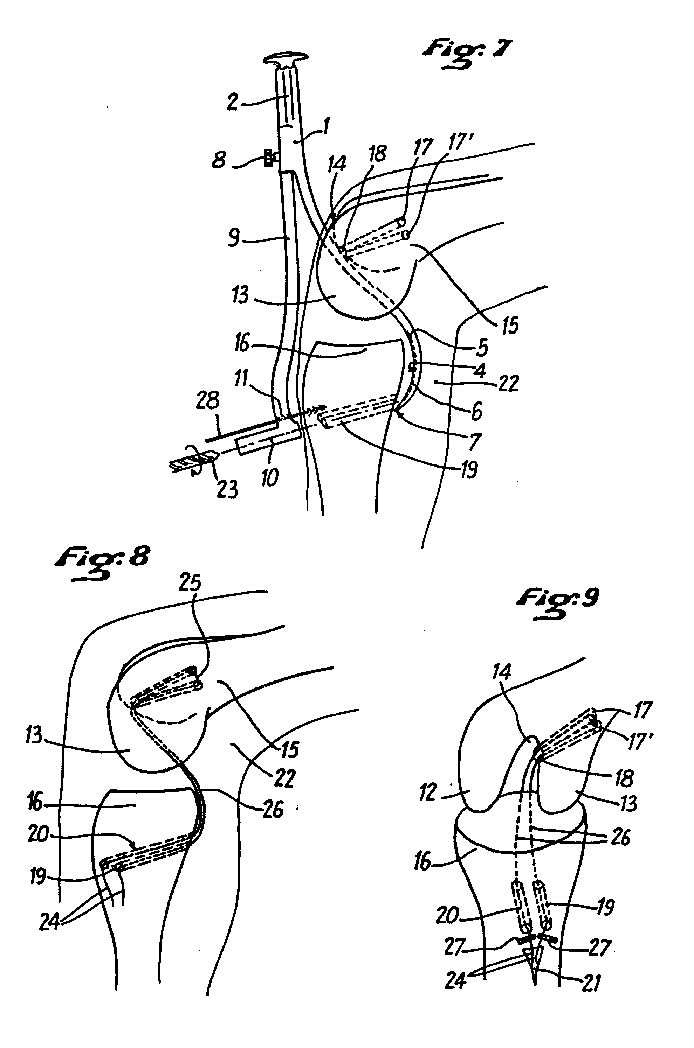

Conformément aux figures 2 et 3, la gouge 1 présente une lame dont les courbures 3 et 4 ont été rigoureusement étudiées, d'une part, pour pénétrer au travers de l'échancrure inter-condylienne 14 montrée dans les figures 6 et suivantes et, d'autre part, descendre le long de la face postérieure du tibia 16 en épousant sensiblement la forme anatomique dudit tibia.

La gouge 1 est par ailleurs munie terminalement d'un bord tranchant 7 permettant de désinsérer, par raclage, tous les éléments attachés à la face postérieure du tibia 16 sur une hauteur de 40 mm environ à compter du bord supérieur du tibia, la descente de la lame étant contrôlée par un repère 5 gravé transversalement à l'intérieur de la partie concave interne 4 de ladite lame ; ce repère 5 est facilement visualisable par l'opérateur au travers de l'échancrure fémorale 14.

Par ailleurs, s'étendant longitudinalement à l'intérieur de la partie concave 4 de la lame, sensiblement depuis le bord tranchant 7 où ils viennent mourir, jusqu'à une distance de 10 mm environ au-dessus du repère 5, deux sillons parallèles sont gravés assez largement pour assurer le guidage sûr d'une broche souple, comme il sera décrit plus loin.

Ainsi constituée, la gouge 1 est descendue conformément à la figure 6 le long de la face postérieure du tibia, le bord 7 en appui sur ladite face et la lame légèrement décollée de la face postérieure du tibia, dégageant ainsi une zone de travail facilitant l'insertion ultérieure du LCP.

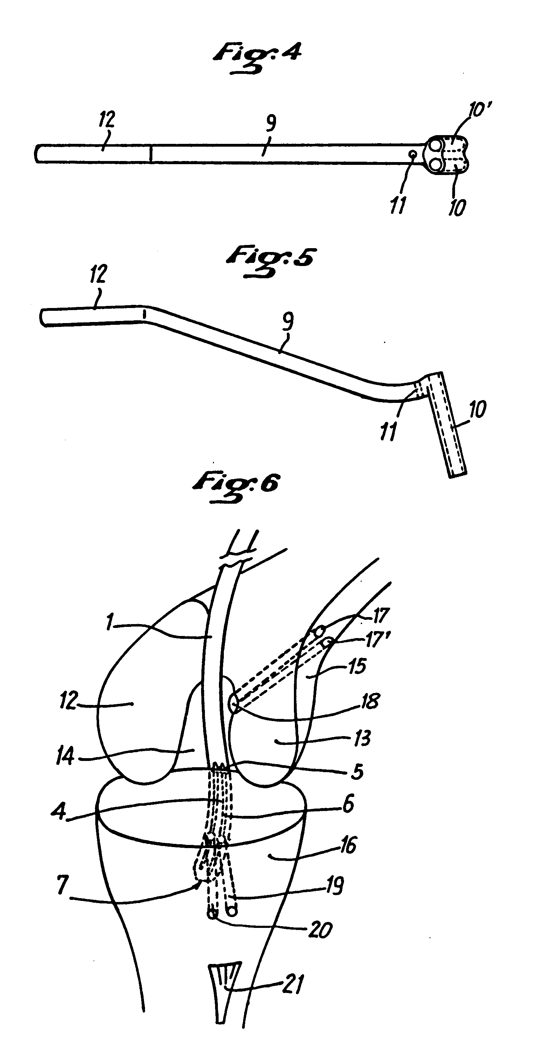

Conformément aux figures 4 et 5, le dispositif de visée 9 comporte un bras de forme étudiée pour épouser le profil anatomique de la face antérieure du tibia et supportant, en bout, deux canons de visée 10 et 10′ accolés, montés de telle sorte que, lorsque le dispositif de visée 9 est fixé à la gouge 1, les axes desdits canons 10 et 10′ aboutissent chacun dans un sillon 6 et 6′ de la face 4 de la lame de ladite gouge 1, suivant une obliquité verticale de 30° environ (angle b).

Dans cette disposition, l'angle a formé par le prolongement de l'axe de chacun des canons et la tangente à la face concave 4 de la lame en leur point d'intersection est de l'ordre de 130°. Les canons 10 et 10′ sont accolés à une distance d'axes de 8 mm et présentent une section de passage utile de diamètre 5,5mm. Par ailleurs, la longueur desdits canons est de 50 mm assurant ainsi un guidage parfait pour les forets 23 venant réaliser les tunnels trans-tibiaux destinés à recevoir les faisceaux du LCP.

Le dispositif de visée 9 est muni d'une queue terminale 12 de section carrée venant, au moment adéquat, s'emboîter dans la rainure 12′ de même section, s'étendant entre le manche 2 et la lame de la gouge 1. Un oeillet 11 de faible diamètre est avantageusement prévu dans le bras du dispositif de visée 9, juste au-dessus des canons de visée 10 et 10′, permettant de loger une broche en acier 28 venant se planter dans l'os pour assurer ainsi le maintien de l'ensemble de l'instrument chirurgical mis en place conformément à la figure 7.

Dès lors, on procède à la perforation des deux tunnels 19 et 20 traversant de part en part le tibia 16. Lorsque le foret 23 débouche dans la face postérieure du tibia, il se trouve en butée sur la partie concave 4 de la lame de gouge 1 évitant ainsi de sérieures blessures à la masse vasculaire postérieure. Les deux tunnels 19 et 20 trans-tibiaux étant effectués, on retire les forets 23 en laissant bien en place tout l'instrument chirurgical suivant l'invention. On peut alors procéder au remplacement prothétique du LCP du genou par le seul abord antérieur.

Les faisceaux 24 du ligament préalablement repliés en forme de "U" sont passés au travers de deux tunnels 17 et 17′ préalablement perforés dans le condyle fémoral interne 13 et débouchant sur la joue condylienne 15 en extra-synovial. Les deux tunnels 17 et 17′ présentent, en outre, la particularité de posséder une entrée commune 18 pratiquée dans l'échancrure fémorale 14 au point anatomique d'insertion fémorale du LCP et des orifices de sortie distincts 17 et 17′, distants entre eux d'au moins 10 mm.

Conformément aux figures 8 et 9, la partie repliées 25 du LCP formant le fond du "U", est placée entre les orifices 17 et 17′ assurant ainsi une fixation immédiate du LCP sur la partie fémorale 15. Les deux brins 26 du LCP débouchant dans l'échancrure 14, sont alors mis en attente. Par les tunnels transtibiaux 19 et 20, on enfile, par la face antérieure du tibia 16, deux broches souples munies terminalement de chas qui viennent tomber dans la partie concave 4 de la lame de gouge 1 avec la seule possibilité de remonter la lame le long des sillons 6 et 6′, étant donné l'angle d'attaque a de 130° environ et ce de telle sorte que lesdites broches souples apparaissent au-delà du repère 5 de la lame, dans l'intervalle intra-articulaire procurant l'avantage d'arrimer les deux brins 26 en attente sur les chas des deux broches et, par retour arrière des équipages ainsi formés, faire apparaître les extrémités 24 du LCP sur la face antéro-interne du tibia. Le tibia 16 est alors maintenu en position de tiroir antérieur à 90°. Des petits greffons cortico-spongieux sont prélevés sur la face antérieure du tibia au droit des tunnels 19 et 20, et enfoncés dans lesdits tunnels pour assurer la fixation immédiate du LCP. Les deux extrémités 24 du LCP sont suffisamment tendues en position de tiroir postérieur à 0, puis amarrées par deux agrafes chirurgicales 27 de 6 mm.

Les reliquats des extrêmités 24 dépassant les agrafes sont enfouis dans la zone de prélèvement 21 des greffons qui est ensuite recouverte par les tendons de la "patte d'oie" qui sont réinsérés à leur position d'origine.

La plastie du LCP du genou effectuée avec l'instrument objet de l'invention procure aussi l'avantage de soins post-opératoires réduits; en particulier aucune immobilisation plâtrée n'est requise. L'instrument permet la mise en place simultanée de deux faisceaux ligamentaires conférant ainsi au montage une solidité immédiate.

## Revendications

1.  Instrument chirurgical de visée et de pose pour la plastie (ou remplacement prothétique) du ligament croisé postérieur du genou comportant une partie qui puisse être introduite au travers de l'échancrure fémorale et un dispositif de visée, caractérisé en ce que pour permettre d'effectuer cette chirurgie par un seul abord antérieur, tout en obtenant un point de pénétration anatomique dudit LCP à la face postérieure du tibia et éviter toute blessure des éléments postérieurs notamment vasculaires l'instrument comprend, d'une part, une gouge composée d'un manche (2) et d'une lame (1) en forme de rugine en "S" italique, dont la partie terminale interne (4) est de section concave, telle que ladite lame (1) puisse être introduite au travers de ladite échancrure fémorale (14) jusqu'à la face postérieure du tibia (16), et d'autre part, ledit dispositif de visée (9), qui vient se solidariser à la gouge (1,2) par des moyens de fixation rapide (8), et comprend à son extrémité, disposés en regard et à distance de la partie concave (4) de la lame (1), deux canons de visée (10) accolés en direction de ladite partie concave (4) suivant une obliquité telle que l'angle (a) formé par le prolongement de l'axe des canons et la tangente à la face interne de la partie concave (4) de la lame (1), en leur point de rencontre, soit supérieur à 90°, la longueur desdits canons de visée (10) étant suffisante pour assurer le guidage longitudinal de forets (23) utilisés pour la perforation transversale de deux tunnels (19,20) dans la partie tibiale (16), ceci permettant la mise en place simultanée de deux faisceaux (24) postéro-interne et postéro-externe du LCP.

2. Instrument chirurgical suivant la revendication 1 caractérisé en ce que la gouge (1) est munie, dans sa partie terminale, d'un bord tranchant en forme de biseau (7) permettant de désinsérer, par raclage de haut en bas, les éléments attachés à la face postérieure du tibia (16).

3. Instrument chirurgical suivant l'une quelconque des revendications précédentes caractérisé en ce qu'à l'intérieur de la partie concave (4) de la lame (1) un repère (5) est gravé transversalement, sur ladite lame (1), à une distance de 40 mm du bord tranchant (7) permettant d'assurer le positionnement précis de la gouge par coïncidence dudit repère (5) avec le bord supérieur du tibia (16) visualisé par l'opérateur dans l'échancrure intercondylienne (14).

4. Instrument chirurgical suivant l'une quelconque des revendications précédentes caractérisé en ce que deux sillons gravés (6,6') parallèles s'étendent longitudinalement à l'intérieur de la partie concave (4) de la lame (1) sensiblement depuis le bord tranchant (7) jusqu'à une distance d'environ 10 mm au-dessus du repère (5), assurant le guidage de broches souples en forme de boucles, du type "passe-lacet", qui viennent remonter dans la zone intra-articulaire pour saisir et mettre en place les deux faisceaux (24) du LPC dans les tunnels (19,20) perforés dans la partie tibiale (16) par simple "retour arrière" desdites broches "passe-lacet".

5. Instrument chirurgical suivant la revendication 4 caractérisé en ce que les deux canons (10,10') en bout du dispositif de visée (9) sont disposés en sorte que l'axe (F) de chacun de ces canons aboutisse dans un sillon (6,6') de la lame (1) en présentant une obliquité verticale (b) d'environ 30° par rapport à l'horizontale, suffisante pour que les broches souples "passe-lacet", traversant les tunnels (19) et (20) dans le tibia (16), abordent, en la remontant, la partie concave (4) de la lame (1), sans buter.

6. Instrument chirurgical suivant l'une quelconque des revendications précédentes caractérisé en ce qu'on oeillet (11) est percé au bas du bras du dispositif de visée (9) portant les canons de visée (10,10'), d'axe colinéaire aux axes desdits canons, permettant d'enfiler une broche d'acier (28) destinée à maintenir l'instrument chirurgical durant toute la plastie du LCP.

**Claims**

1. Surgical instrument for positioning and inserting for plasty (or prosthetic replacement) of the posterior cruciate ligament of the knee, comprising a part which may he introduced across the femoral notch and a positioning device (visor), characterized in that, in order to allow this surgery to be carried out by a sole anterior approach, whilst obtaining an anatomical point of penetration of said PCL at the posterior face of the tibia and avoiding any damage of the posterior elements, notably vascular, the instrument comprises, on the one hand, a chisel composed of a handle (2) and a blade (1) in the form of an italic "S" rugine, of which the internal terminal part (4) is concave in section, such that said blade (1) can be introduced across said femoral notch (14) up to the posterior face of the tibia (16), and, on the other hand, said visor device (9) which is anchored on the chisel (1, 2) by rapid fixation means (8), and comprises ar its end, arranged in relation to and at a certain distance from the concave part (4) of the blade (1), two visor branches (10) joined together in the direction of the said concave part (4) following an oblique direction such that the angle (a) formed by the elongation of the axis of the visor branches and the tangent to the internal face of the concave part (4) of the blade (1), at their meeting point, is greater than 90°, the length of said visor branches (10) being sufficient to ensure longitudinal guiding of drills (23) used for the transverse perforation of two tunnels (19, 20) in the tibial section (16), thus permitting the simultaneous insertion of two postero-internal and postero-external parts (24) of the PCL.

2. Surgical instrument according to Claim 1, characterized in that the chisel (1) is fitted, in its terminal part, with a cutting edge in the shape of a bevel (7) enabling dissection, by scraping from top to bottom, of the elements attached to the posterior face of the tibia (16).

3. Surgical instrument according to either one of the preceding Claims, characterized in that, inside the concave part (4) of the blade (1), a mark (5) is engraved transversely on said blade (1) at a distance of 40 mm from the cutting edge (7) ensuring the precise positioning of the chisel by its coincidence with the said mark (5) with the upper edge of the tibia (16) visualized by the operator in the intercondylar notch (14).

4. Surgical instrument according to any one of the preceding Claims, characterized in that two parallel engraved grooves (6, 6') extend longitudinally inside the concave part (4) of the blade (1) substantially from the cutting edge (7) up to a distance of about 10 mm above mark (5), eisuring guiding of supple pins in the form of buckles, of the "bodkin" type, which rise up in the inter-articular zone in order to grasp and position the two parts (24) of the PCL in the tunnels (19, 20) perforated in the tibial section (16) by simple "rearward return" of said "bodkin" pins.

5. Surgical instrument according to Claim 4, characterized in that the two visor branches (10, 10') at the end of the visor device (9) are disposed so that the axis (F) of each of them culminates in a groove (6, 6') of the blade (1), presenting a vertical obliqueness (b) of about 30° with respect to the horizontal, sufficient for the supple "bodkin" pins, traversing the tunnels (19) and (20) in the tibia (16), to approach, rising up, the concave section (4) of the blade (1), without abutting.

6. Surgical instrument according to any one of the previous Claims, characterized in that a hole (11) is pierced at the bottom of the arm of the visor device (9) bearing the visor branches (10, 10'), whose axis is colinear to the axes of said branches, making it possible to thread a steel pin (28) intended to hold the surgical instrument throughout the PCL plasty.

**Patentansprüche**

1. Chirurgisches Instrument zum Positionieren und Befestigen für eine Plastik (oder Ersatzprothese) des hinteren Kreuzbandes (LCP) des Kniegelenks, mit einem Teil, das quer zur femoralen Einbuchtung einführbar ist, und mit einer Ziel-oder Positioniervorrichtung, **dadurch gekennzeichnet, daß** das Instrument, um die Durchführung dieser Operation durch einen einzigen vorderen Zugang zu ermöglichen, alles indem man einen anatomischen Durchstoßpunkt des LCP an der hinteren Seite der Tibia erzielt und jede Verletzung von hinteren Elementen, insbesondere Gefäßen vermeidet, einerseits einen Hohlmeißel aus einem Griff (2) und einer Klinge (1) in Form einer schrägen, S-förmigen Knochenfeile, deren inneres Endteil (4) einen konkaven Querschnitt hat, so daß die Klinge (1) quer durch die erwähnte femorale Einbuchtung (14) bis zur hinteren Seite der Tibia (16) eingeführt werden kann, und andererseits die erwähnte Positioniervorrichtung (9) enthält, welche mit dem Hohlmeißel (1, 2) durch eine Schnellbefestigungsvorrichtung (8) verbunden ist und an ihrem Ende, welches sich im Abstand vom konkaven Teil (4) der Klinge (1) befindet, zwei Positionierröhren (10) aufweist, die mit einer solchen Schrägstellung in der Richtung auf das besagte konkave Teil (4) angebracht sind, daß der Winkel (a), der durch die Verlängerung der Achse der Röhren und die Tangente an die Innenfläche des konkaven Teiles (4) der Klinge (1) am Schnittpunkt gebildet wird, größer als 90° ist, wobei die Länge der besagten Positionierröhren (10) genügend groß ist um eine Längsführung der Bohrer (23) zu gewährleisten, die für die transversale Perforation von zwei Kanälen (19, 20) im Tibiateil (16) verwendet werden, welche die gleichzeitige Befestigung der beiden Stränge (24) des LCP, des inneren hinteren und des äußeren hinteren, ermöglichen.

2. Chirurgisches Instrument nach Anspruch 1, **dadurch gekennzeichnet, daß** der Hohlmeißel (1) an seinem Endteil mit einer Schneidekante in Form einer Facette (7) versehen ist, die es erlaubt, durch Schaben von oben nach unten Elemente, die sich an der hinteren Seite der Tibia (16) befinden, zu entfernen.

3. Chirurgisches Instrument nach einem der vorhergehenden Ansprüche, **dadurch gekennzeichnet, daß** im Inneren des konkaven Teiles (4) der Klinge (1) eine Markierung (5) quer auf der Klinge (1) in einem Abstand von 40 mm von der Schneidkante (7) eingraviert ist, welche eine exakte Positionierung des Hohlmeißels durch Zusammentreffen der Markierung (5) mit dem oberen Rand der Tibia (16), den der Chirurg in der interkondylen Einbuchtung (14) sieht, gewährleistet.

4. Chirurgisches Instrument nach einem der vorhergehenden Ansprüche, **dadurch gekennzeichnet, daß** sich zwei parallele gravierte Rillen (6, 6') innen auf dem konkaven Teil (4) der Klinge (1) in Längsrichtung im wesentlichen von der Schneidkante (7) bis ungefähr 10 mm über die Markierung (5) hinaus erstrecken, welche die Führung von weichen Dornen in Form von Schlaufen vom Typ "Schnürnadel" gewährleisten, welche man in der intraartikularen Zone austreten läßt, um die beiden Stränge (24) des LPC in den Kanälen (19, 20), die in den Tibiateil (16) eingebracht wurden, durch einfaches Hin- und Herbewegen der "Schnürnadel"-Dorne zu positionieren.

5.  Chirurgisches Instrument nach Anspruch 4, **dadurch gekennzeichnet,** daß die beiden Röhren (10, 10') am Ende der Positioniervorrichtung (9) so angeordnet sind, daß die Achse (F) jeder dieser Röhren in eine Rille (6, 6') der Klinge (6) eintritt, wobei sie einen vertikalen Winkel (b) von etwa 30° bezüglich der Horizontalen bildet, der ausreicht, um die weichen "Schnürnadel"-Dorne, die die Kanäle (19) und (20) in der Tibia (16) durchsetzen, beim Einschieben am konkaven Teil (4) der Klinge (1) ohne aufzustoßen hochsteigen zu lassen.

6.  Chirurgisches Instrument nach einem der vorhergehenden Ansprüche, **dadurch gekennzeichnet,** daß ein Ösenloch (11) unten am Arm der Positioniervorrichtung (9), der die Positionierröhren (10, 10') trägt, vorgesehen ist, dessen Achse parallel zu den Achsen der Röhren verläuft, um das Einführen eines Stahldornes (28) zu ermöglichen, der dazu dient, das chirurgische Instrument während der ganzen Plastik des LCP zu halten.

*Fig: 1*

*Fig: 2*

*Fig: 3*

*Fig. 4*

*Fig. 5*

*Fig. 6*

Fig: 7

Fig: 8

Fig: 9